(19)

Europäisches
Patentamt
European
Patent Office
Office européen
des brevets

(11) **EP 4 134 030 A1**

(12) **EUROPEAN PATENT APPLICATION**

(43) Date of publication:
**15.02.2023   Bulletin 2023/07**

(21) Application number: **22189404.1**

(22) Date of filing: **09.08.2022**

(51) International Patent Classification (IPC):
**A61B 18/12** (2000.01)          **A61B 18/14** (2000.01)
**A61B 18/00** (2000.01)

(52) Cooperative Patent Classification (CPC):
**A61B 18/12; A61B 18/14; A61B 18/1492;**
A61B 2018/00404; A61B 2018/00434;
A61B 2018/00577; A61B 2018/00613;
A61B 2018/1407; A61B 2018/1467;
A61B 2018/1475

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.08.2021   US 202163231639 P
15.07.2022   US 202217866160**

(71) Applicant: **Biosense Webster (Israel) Ltd
Yokneam, 2066717 (IL)**

(72) Inventors:
• **GOVARI, Assaf
  Yokneam 2066717 (IL)**
• **ALTMANN, Andres Claudio
  Yokneam 2066717 (IL)**

(74) Representative: **Carpmaels & Ransford LLP
One Southampton Row
London WC1B 5HA (GB)**

(54)     **A METHOD FOR RENAL DENERVATION**

(57)     A catheter, consisting of an insertion probe, having a distal end configured for insertion into a lumen of a human subject. The catheter also has a resilient tube, extending distally from the distal end of the insertion probe and having, when unconstrained, a planar serpentine shape contained within a plane that contains a longitudinal axis of the distal end of the insertion probe. A plurality of electrodes are fixedly attached to the resilient tube and are configured to transfer ablation energy to the human subject. Other shapes for the insertion tube are also provided.

**EP 4 134 030 A1**

**Description**

**CROSS-REFERENCE TO RELATED APPLICATION**

This application claims the benefit of U.S. Provisional Patent Application 63/231,639, filed August 10th, 2021, which is incorporated herein by reference.

**FIELD OF THE DISCLOSURE**

The present disclosure relates generally to renal denervation, and specifically to denervation by ablation performed using electrical energy.

**BACKGROUND**

Renal denervation is a process whereby nerves in the wall of the renal artery are ablated, and it may be used as a treatment for resistant hypertension.
Ablation of tissue may be performed using radiofrequency (RF) electrical energy, which destroys the cells of the tissue by overheating the tissue. The cell death from RF ablation, necrosis, results in the destruction of the cell through the action of their own enzymes.
Alternatively, tissue may be ablated using irreversible electroporation (IRE). IRE is a tissue ablation technique that applies short pulses of strong electrical fields to create permanent and hence lethal nanopores in the cell membrane, thus disrupting the cellular homeostasis (internal physical and chemical conditions). Cell death following IRE results from apoptosis (programmed cell death) and not from necrosis. IRE is commonly used in tumor ablation in regions where precision and conservation of the extracellular matrix, blood flow and nerves are of importance.
The present disclosure will be more fully understood from the following detailed description of the examples thereof, taken together with the drawings, in which:

**BRIEF DESCRIPTION OF THE DRAWINGS**

Fig. 1 is a schematic illustration of an ablation system; and
Figs. 2A - 2D are respective schematic diagrams of resilient tubes of a catheter.

**DESCRIPTION OF EXAMPLES**

Overview

In order to be effective, both IRE (irreversible electroporation) and RF (radiofrequency) ablation modalities require that the electrodes used to transfer the electrical energy are configured so as not to be damaged by the transfer. Examples of the disclosure achieve this by forming the electrodes to be relatively massive, and also by irrigating the electrodes, using irrigation channels within the electrodes, so that the electrodes do not overheat. The electrodes are formed on a flexible resilient tube, typically having an approximate circumference equal to 8 French, so that conductive wires and irrigation channels to the electrodes may be positioned within the tube. The resilient tube, with its electrodes, is configured as extending distally from the distal end of an insertion probe of a catheter used to perform renal denervation. In some examples, the electrodes configured for ablating may also be operated as part of a tracking system for tracking electrode and/or resilient tube location in-vivo. In some examples, the resilient tube includes one or more electrodes dedicated for tracking, e.g. dedicated for being operated as part of the tracking system for in-vivo tracking.
The renal denervation is performed by positioning the resilient tube in the renal artery, or alternatively in the renal vein. Access to the renal artery is via the femoral artery, and initially the surgeon performing the denervation translates a sheath through the femoral artery until the sheath exit is at the opening to the renal artery. The surgeon translates the catheter resilient tube through the sheath, in a form constrained by the sheath, and the tube expands into its unconstrained form when it exits from the sheath. In the disclosure, the shape of the resilient tube in its unconstrained form is assumed to be the shape of the tube when the only external force acting thereon is the force of gravity.
Typically, the resilient tube is constrained to have a more narrow and elongated shape while within the sheath. As the resilient tube is extracted from the sheath, it begins to bend and contract to transition from its constrained shape to its operative shape. An operative shape of the resilient tube as defined herein is the shape of the resilient tube in the body lumen after being pushed out through the sheath. The constrained shape of the resilient tube as defined herein is the shape of the resilient tube inside the sheath.

The inventors have found that forming the unconstrained resilient tube in one of a number of predefined shapes facilitates controlling movement of the resilient tube as it comes out of the sheath and advances through a tortuous path of the renal artery. Thus, examples of the disclosure form the unconstrained resilient tube into one of the following predefined shapes:

- A planar two-dimensional shape.
- A helical spiral that is no more than one complete spiral.
- A simple, relatively straight, wire.

Forming the unconstrained resilient tube in one of these shapes makes it possible to control the movement of the distal tip of the tube as it exits from the sheath, since there is no, or very little, change in shape between the constrained and unconstrained shape of the resilient tube. This is in contrast to prior art systems, where the change of shape of the distal end of a catheter as it exits the sheath makes it difficult to control the distal end movement.

The present inventors have found that when attempting to push a distal end out of a sheath and into a tortuous path of a blood vessel, the distal end may turn in on itself as it transitions from its constrained shape to its operational shape. Once the distal tip begins to fold and/or turn into itself it may be difficult if not impossible to advance the distal tip through the blood vessel in a controlled manner. The present inventors have found that this often occurs when the distal tip has an unconstrained shape such as a long 3D spiral shape or the like. The present inventors have found that such occurrences may be avoided or reduced based on using a flatter geometry for the distal tip. The present inventors have found that by using a flatter and/or more simplified configuration as described herein, the physician may have better control of the movement of the distal tip through the body lumen, e.g. blood vessel as it emerges out of the sheath.

System Description

In the following description, like elements in the drawings are identified by like numerals, and like elements are differentiated as necessary by appending a letter to the identifying numeral.

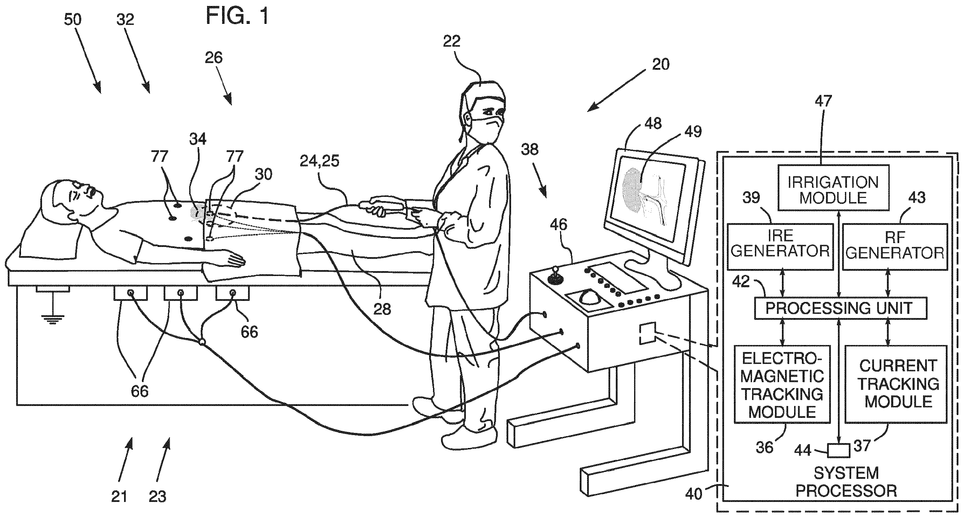

Reference is now made to Fig. 1, which is a schematic illustration of an ablation system 20, according to an example of the present disclosure. For simplicity and clarity, the following description, except where otherwise stated, assumes a medical procedure is performed by a user 22 of system 20, herein assumed to be a medical practitioner, wherein the user inserts a catheter 24, also herein termed probe 24, into a left or right femoral artery of a patient 28. Probe 24 is a tubular element, defining a central lumen 25. User 22 is also referred to herein as operator 22. The procedure is assumed to comprise renal denervation of nerves in a renal artery 34 of the patient, and in the procedure, the probe is initially inserted into the patient until a resilient tube, extending distally from the distal end of the probe reaches the renal artery. As is described in more detail below, the present disclosure provides different examples of the resilient tube, which is hereinbelow generically termed resilient tube 32, and for clarity the different examples of the resilient tube are termed resilient tube 32A, 32B, 32C, ....

In examples of the invention, resilient tube 32 may have an outer diameter in the range of approximately 1 mm to approximately 3 mm. In a disclosed example the range is approximately 1.5 mm to approximately 3 mm.

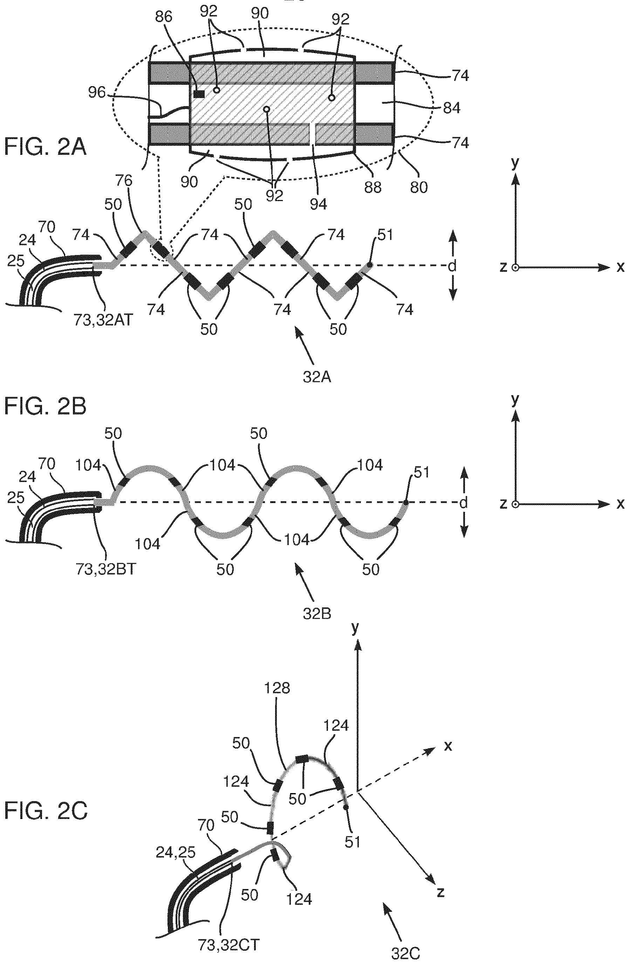

Typically, to facilitate the insertion of catheter resilient tube 32, a tubular sheath 70 (described in more detail with respect to Figs. 2A - 2D below) is first inserted into the femoral artery and the sheath is translated within the artery until the distal end of the sheath is proximate to the renal artery. Catheter 24 is then inserted into the sheath, as stated above.

System 20 may be controlled by a system processor 40, comprising a processing unit (PU) 42 communicating with an electromagnetic tracking module 36 and/or a current tracking module 37, as well as with an irrigation module 47. PU 42 also communicates with an IRE (irreversible electroporation) generator 39 and/or an RF (radiofrequency) generator 43. The functions of the modules and the generators are described in more detail below. PU 42 also communicates with a memory 44. Processor 40 is typically mounted in a console 46, which comprises operating controls 38, typically including a pointing device such as a mouse or trackball, that operator 22 uses to interact with the processor. The processor uses software stored in memory 44 to operate system 20, and results of the operations performed by processor 40 are presented to the operator on a display 48. The software may be downloaded to processor 40 in electronic form, over a network, for example, or it may, alternatively or additionally, be provided and/or stored on non-transitory tangible media, such as magnetic, optical, or electronic memory.

For tracking the path of resilient tube 32 in a mapping region 30 containing renal artery 34, examples of the present disclosure use at least one of a current based tracking system 21 and an electromagnetic based tracking system 23. Both systems are described below.

Tracking system 21 comprises a current measuring tracking system, similar to that described in US Patent 8,456,182 to Bar-Tal et al., whose disclosure is incorporated herein by reference. The Carto® system produced by Biosense-Webster of 33 Technology Drive, Irvine, CA 92618 USA, also uses a current measuring tracking system. The current measuring tracking system is under control of current tracking module 37. As is described in more detail below, resilient tube 32 has a plurality of electrodes 50, and in tracking system 21 module 37 injects currents to the electrodes 50 being tracked. The currents are received, by a plurality of generally similar patch electrodes 77, also herein termed patches, which are positioned on the skin of patient 28 in proximity to artery 34, and transferred back to the module.

While conductive cabling to patch electrodes 77 described herein is present for each of the electrodes, for clarity cabling is only shown in the figure for some of the electrodes. The currents between a given resilient tube electrode 50 and skin patches 77 vary according to the location of the electrode, because, *inter alia,* of the different distances of the electrode from the patches, which cause different impedances between the given resilient tube electrode and the different patches. Module 37 measures the different currents received by the different patches 77 on respective channels connected to the patches, and may be configured to generate an indication of the location of the given resilient tube electrode from the different currents.

Electromagnetic tracking system 23 is similar to that described in US Patent 6,690,963 to Ben-Haim et al., whose disclosure is incorporated herein by reference, and to that used in the Carto® system. The electromagnetic tracking system is under control of electromagnetic tracking module 36. The electromagnetic tracking system comprises a plurality of magnetic field generators, herein assumed to comprise three sets of generators 66, each set comprising three orthogonal coils, so that the plurality of generators comprises a total of nine coils. Generators 66 are placed in known locations beneath patient 28, the known locations defining a frame of reference of the generators. Module 36 controls, *inter alia,* the amplitude and frequency of the alternating magnetic fields produced by the generators.

The alternating magnetic fields interact with a sensor 51 (described with respect to Figs. 2A - 2D below) located in resilient tube 32, so as to generate alternating electropotentials in the sensor, and the electropotentials are received as a signal by tracking module 36. The module, together with processing unit 42, analyzes the received signal, and from the analysis is able to determine a position, i.e., a location and an orientation, of the resilient tube d coil in the defined frame of reference.

Typically the tracking by either or both of the systems may be presented visually on display 48, for example by incorporating an icon representing the resilient tube into a map 49 of renal artery 34 and its surroundings, as well as a path taken by the icon. For clarity, in the following description, only electromagnetic tracking system 23 is assumed to be used, but the description may be adapted, *mutatis mutandis,* for cases where both system 23 and system 21 are used, or if only system 21 is used.

To provide ablation energy to electrodes 50 on probe 24, PU 42 also uses a radiofrequency (RF) generator 43 and/or an irreversible electroporation (IRE) Generator 39. RF generator 43 is configured to supply alternating power of up to approximately 100 W at a frequency of the order of approximately 100 kHz. The RF power is supplied to individual electrodes 50 selected by user 22, so as to destroy tissue receiving the power by necrosis. A generator similar generator 43 is provided in the Carto® system of Biosense-Webster.

IRE generator 39 is configured to supply alternating power in pulses at frequencies of up to approximately 1 MHz, and with pulse amplitudes of up to approximately 2 kV, and at currents up to approximately 20 A. The pulses are typically supplied to electrodes 50 which are selected by PU 42 according to a preset protocol accessed by user 22. The pulses are supplied in bursts of trains of pulses - a train comprising a set of up to 100 pulses, and a burst comprising up to 100 trains. The bursts are configured, and the electrodes receiving the bursts are selected, so that tissue in proximity to the electrodes is destroyed by apoptosis. A detailed description of an IRE generator similar to generator 39 is provided in U.S. Patent Application 16/701,989, titled "Pulse Generator for Irreversible Electroporation," which is incorporated herein by reference.

Electrodes 50 are irrigated, and PU 42 controls the rate of irrigation using irrigation module 47 and signals from temperature sensors in the electrodes. The rate is set so that electrodes 50 are effectively cooled while they are transferring ablation energy, and is typically of the order of 60 mL/minute, although the rate may be larger or smaller than this rate.

Figs. 2A - 2D are respective schematic diagrams of resilient tubes 32A, 32B, 32C, 32D, according to examples of the present disclosure. Each of the figures illustrates a resilient tube 32 after it has emerged from sheath 70, and in each figure the resilient tube is drawn on a set of xyz Cartesian axes, where an x-axis is collinear with a symmetry axis of the exit of the sheath. In Figs. 2A and 2B, within sheath 70 probe 24 and its internal lumen 25 are shown schematically.

As stated in the procedure above, once a given resilient tube 32 emerges from sheath 70, it enters the renal artery, which typically constrains the shape of the resilient tube. It will be understood that the figures show resilient tube 32 outside the sheath and in an unconstrained state.

An overall length of each of the resilient tubes described below is approximately 5 cm although the overall length may be larger or smaller than this value.

Fig. 2A illustrates resilient tube 32A, which comprises a flexible tube 76 shaped to include two or more segments 74 formed from a plastic, such as polyurethane or polyamide, or from nitinol. A proximal termination 32AT of resilient tube 32A is fixedly coupled to a distal end 73 of probe 24 and segments 74 are joined together so that the resilient tube d is one integral element, forming a single linear entity which lies in a two-dimensional plane. The regions where the segments are joined together, i.e., the "corners" of the resilient tube, may be curved. As illustrated in the figure, resilient tube 32A lies in an xy plane, where the x-axis and the y-axis are in the plane of the paper, and the z-axis is orthogonal to the paper. The x-axis is, as stated above, collinear with a symmetry axis of the exit of sheath 70 and this symmetry axis is also assumed to correspond to a longitudinal axis of probe 24 at distal end 73. In a disclosed example segments 74 make an angle of approximately 45° with the x-axis, although this angle may be larger or smaller than 45°. In some examples, segments 74 may be arranged so that resilient tube 32A is symmetrical with respect to the x-axis, as is illustrated in the figure, and so that a peak-peak distance d measured orthogonal to the x-axis is in a range of approximately 2 mm to approximately 6 mm.

In a disclosed example sensor 51, which may comprise a single axis sensor (SAS), a dual axis sensor (DAS), or a triple axis sensor (TAS), is formed on a distal termination of resilient tube 32A. Alternatively, sensor 51 may be located along the length of the tube. The possible locations of sensor 51, i.e. at the distal termination of the resilient tube or along the length of the tube, apply to the other examples of resilient tube 32 described herein.

Wires from the sensor are coupled to processor 40, so that current tracking module 37 is able to provide a location of the distal termination of resilient tube 32A using currents generated in the sensor. Alternatively or additionally, resilient tube 32A may be tracked by currents between electrodes 50 and patches 77, as is described above.

Each electrode 50 is mounted on a given segment 74, and the electrodes, typically formed from platinum-iridium or gold, are configured to transfer ablation current from IRE generator 39 and/or RF generator 43 to tissue of the renal artery. A callout 80 is a schematic cross-section illustrating the structure of electrode 50 and segment 74, and the callout illustrates that every tubular segment 74 defines an internal lumen 84. It will be understood that since segments 74 are joined together, lumen 84 forms a continuous lumen within all of the segments, i.e., within tube 76.

One or more of electrodes 50 has a temperature sensor 86 which is coupled to irrigation module 47. PU 42 and module 47 use signals from sensors 86 to control the irrigation flow to electrodes 50.

Each given electrode 50 is formed to have cylindrical symmetry. In a disclosed illustrated example, electrode 50 has an ellipsoidal outer shell 88 with a longitudinally extending bore, and the electrode is fixed around tubular segment 74. The outer ellipsoidal surface of shell 88 increases the surface area of the electrode (compared to a cylinder), while being atraumatic. Between shell 88 and the outer surface of tube 74 there is a generally toroidal volume 90, and a channel 94 connects volume 90 to lumen 84.

Within each electrode there are a plurality of irrigation holes 92, which penetrate shell 88. Consequently, irrigation fluid provided by irrigation module 47 is able traverse lumen 25, lumen 84, and channel 94 so as to enter volume 90, and from there irrigate and cool every electrode 50 of the resilient tube through respective holes 92. It will be understood that absent such cooling, any electrode 50 may be irreversibly damaged by the currents from IRE generator 39 or RF generator 43.

As stated above, currents from IRE generator may be of the order of 20 A, and they are transmitted to electrodes 50 at potentials of up to approximately 2 kV. Each electrode 50 is consequently galvanically connected to processor 40 (and thus to the IRE and the RF generators) by insulated cabling 96, which traverses lumen 35.

Fig. 2B illustrates resilient tube 32B, which comprises a plurality of two or more curvilinear flexible tubular segments 104. Apart from the differences described below, the operation of resilient tube 32B is generally similar to that of resilient tube 32A (Fig. 2A), and elements indicated by the same reference numerals in both resilient tubes are generally similar in construction and in operation. Thus, callout 80, and the description above regarding electrodes 50 as given above with respect to the callout, applies to the description herein for resilient tube 32B. A proximal termination 32BT of resilient tube 32B is fixedly coupled to distal end 73 of probe 24.

As illustrated in the figure, resilient tube 32B comprises two or more curved flexible tubular segments 104 which are joined together so that, as for resilient tube 32A, resilient tube 32B is one integral element, forming a single linear entity which lies in a two-dimensional plane. The single linear entity of resilient tube 32B comprises a plurality of electrodes 50, substantially as described above for resilient tube 32A, and it will be understood that resilient tube 32B also comprises a lumen containing cabling for the electrodes as well as providing a channel for irrigation fluid for the electrodes.

As stated above, both resilient tube 32A and 32B lie in a two-dimensional plane, and from the figures it will be understood that the resilient tubes are single serpentine entities having a peak-peak value between approximately 2 mm and approximately 6 mm. Using the sets of axes of the figures, an expression for the shape of the resilient tubes is:

$$y = f(x); \quad z = c \qquad (1)$$

where f(x) is a function of x (different for tubes 32A and 32B), and
c is a constant. (In the figures, z = 0.)

Fig. 2C illustrates resilient tube 32C, which comprises a plurality of two or more curvilinear flexible tubular segments 124. Apart from the differences described below, the operation of resilient tube 32C is generally similar to that of resilient tubes 32A and 32B, and elements indicated by the same reference numerals in the different resilient tubes are generally similar in construction and in operation. Callout 80, and the description above regarding electrodes 50 as given above with respect to the callout, applies to the description herein for resilient tube 32C. A proximal termination 32CT of resilient tube 32C is fixedly coupled to distal end 73 of probe 24.

As for resilient tubes 32A and 32B, resilient tube 32C is formed as a single linear entity that is one integral element. However, in contrast to resilient tubes 32A and 32B, resilient tube 32C does not lie in a two-dimensional plane. Rather resilient tube 32C is formed as a three-dimensional helix 128. An axis of symmetry of helix 128, herein assumed to be an x-axis, is collinear with a symmetry axis of the exit of sheath 70 and this symmetry axis is also assumed to correspond to a longitudinal axis of probe 24 at distal end 73.

Furthermore, helix 128 is formed to be no more than a single spiral. In some examples an outer diameter d of helix 128 is approximately 5 mm, but other examples may have different outer diameters.

Helix 128 comprises a plurality of electrodes 50, substantially as described above for resilient tubes 32A and 32B.

As stated above, helix 128 is no more than a single spiral, so, for example, one example may comprise half a spiral, and another example may comprise two-thirds of a spiral.

In Fig. 2C, helix 128 has been drawn on a set of xyz Cartesian axes, where the x-axis of the helix is as defined above, and the y and z axes are orthogonal to each other and to the x-axis. Using these axes, an expression for the shape of resilient tube 32C is:

$$y = a \cdot \cos(t); \quad z = a \cdot \sin(t); \quad x = t; \quad 0 < t \le 2\pi \qquad (2)$$

where t is a parameter defining a location of resilient tube 32C with respect to the x-axis, and a is the radius of the helix defined by the equations. (If diameter d = 5 mm, a = 2.5 mm.)

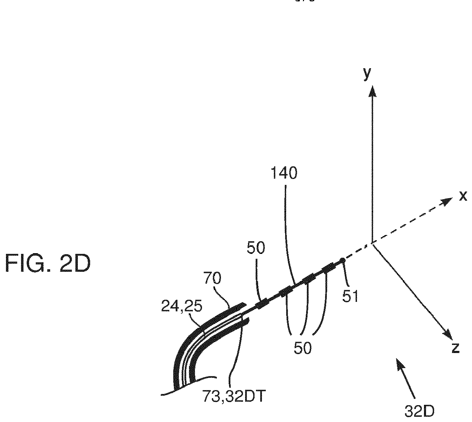

Fig. 2D illustrates resilient tube 32D, which is formed as a single plastic flexible tubular element 140. Apart from the differences described below, the operation of resilient tube 32D is generally similar to that of resilient tubes 32A, 32B, 32C and elements indicated by the same reference numerals in the resilient tubes are generally similar in construction and in operation. Thus, callout 80, and the description above regarding electrodes 50 as given above with respect to the callout, applies to the description herein for resilient tube 32D. A proximal termination 32DT of resilient tube 32D is fixedly coupled to distal end 73 of probe 24.

As for resilient tubes 32A, 32B, and 32C a plurality of electrodes 50 are fixed around element 140, and the element has an internal lumen providing cabling and irrigation to the electrodes. However, in contrast to resilient tubes 32A, 32B, and 32C, resilient tube 32D comprises a single entity that is neither two nor three-dimensional, but is substantially one-dimensional, being in the form of a generally straight line.

In Fig. 2D resilient tube 32D has been drawn on a set of xyz Cartesian axes, where the x-axis of the tube is collinear with a longitudinal axis of probe 24 at distal end 73, and the y and z axes are orthogonal to the x-axis and to each other. Using the axes of Fig. 2D, an expression for the shape of resilient tube 32D is:

$$x = t; \quad y = z = 0 \qquad (3)$$

where t is a parameter corresponding to a location on the x-axis.

As described above for tubes 32, in some examples element 140, i.e., tube 32D, has an outer diameter in a range from approximately 1 mm to approximately 3 mm. In a disclosed example the outer diameter is in a range from approximately 1.5 mm to approximately 3 mm.

Referring back to Fig. 1, renal denervation may be implemented in patient 28 by positioning one of resilient tubes 32 described above in a renal artery, or a renal vein, of the patient. Once a selected resilient tube is so positioned IRE generator 39 and/or RF generator 43 are activated to supply ablation energy to selected electrodes 50 of the resilient tube. The energy may be supplied in a unipolar manner, between a selected electrode 50 and one or more electrodes on the skin of patient 28. Alternatively or additionally, the energy may be supplied in a bipolar manner, between one or more selected pairs of electrodes 50. Typically, in the case of IRE ablation, electrodes for unipolar operation, or selected pairs of electrodes for bipolar operation, are selected in a sequential manner, so as to improve the efficiency of the IRE ablation. Sequential electrode selection is described in U.S. Patent Application 16/701,989 referenced above.

The inventors believe that the renal denervation achieved by examples of the present disclosure is because applying IRE energy and/or RF energy to the resilient tubes as described herein may kill ganglia of the nerves. I.e., rather than killing complete nerves, examples of the present disclosure only kill the ganglia of the nerves.

The description above has assumed that the shape of electrodes 50 is ellipsoidal. However, it will be understood that the scope of the present disclosure includes electrodes having shapes with cylindrical symmetry other than the ellipsoidal shape described above, for example, toroidal or cylindrical shapes, and those having ordinary skill in the art will be able to modify the description, *mutatis mutandis,* for such alternate shapes.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values $\pm 10\%$ of the recited value, e.g. "approximately 90%" may refer to the range of values from 81% to 99%.

EXAMPLES

Example 1. A catheter, comprising:

an insertion probe (24), having a distal end configured for insertion into a lumen of a human subject;
a resilient tube (32A, 32B), extending distally from the distal end of the insertion probe and having, when unconstrained, a planar serpentine shape contained within a plane that contains a longitudinal axis of the distal end of the insertion probe; and
a plurality of electrodes (50), fixedly attached to the resilient tube, configured to transfer ablation energy to the human subject.

Example 2. The catheter according to example 1, wherein the resilient tube comprises a plurality of straight elements coupled together as a single linear entity.
Example 3. The catheter according to example 1, wherein the resilient tube comprises a plurality of curvilinear elements coupled together as a single linear entity.
Example 4. The catheter according to example 1, wherein the planar serpentine shape has a peak-peak value between 2 mm and 6 mm.
Example 5. The catheter according to example 1, wherein the ablation energy comprises irreversible electroporation (IRE) energy provided by an IRE generator.
Example 6. The catheter according to example 1, wherein the ablation energy comprises radiofrequency (RF) energy provided by an IRE generator.
Example 7. The catheter according to example 1, wherein the lumen is one of a renal artery and a renal vein of the human subject.
Example 8. A catheter, comprising:

an insertion probe (24), having a distal end configured for insertion into a lumen of a human subject;
a resilient tube (32C), extending distally from the distal end of the insertion probe and having, when unconstrained, a helical shape having no more than a single spiral and an axis that is collinear with a longitudinal axis of the distal end of the insertion probe; and
a plurality of electrodes (50), fixedly attached to the resilient tube, configured to transfer ablation energy to the human subject.

Example 9. The catheter according to example 8, wherein an outer diameter of the helical shape is 5 mm.
Example 10. A catheter, comprising:

an insertion probe (24), having a distal end configured for insertion into a lumen of a human subject;
a resilient tube (32D), extending distally from the distal end of the insertion probe and having, when unconstrained, a linear shape having an axis that is collinear with a longitudinal axis of the distal end of the insertion probe; and
a plurality of electrodes, fixedly attached to the resilient tube, configured to transfer ablation energy to the human subject.

Example 11. The catheter according to example 10, wherein an outer diameter of the linear shape is in a range from 1 mm to 3 mm.
Example 12. A method, comprising:

providing an insertion probe (24), having a distal end configured for insertion into a lumen of a human subject;
extending a resilient tube (32A, 32B) distally from the distal end of the insertion probe, the resilient tube having, when unconstrained, a planar serpentine shape contained within a plane that contains a longitudinal axis of the distal end of the insertion probe; and

fixedly attaching a plurality of electrodes (50), configured to transfer ablation energy to the human subject, to the resilient tube.

Example 13. The method according to example 12, wherein the resilient tube comprises a plurality of straight elements coupled together as a single linear entity.

Example 14. The method according to example 12, wherein the resilient tube comprises a plurality of curvilinear elements coupled together as a single linear entity.

Example 15. The method according to example 12, wherein the planar serpentine shape has a peak-peak value between 2 mm and 6 mm.

Example 16. The method according to example 12, wherein the ablation energy comprises irreversible electroporation (IRE) energy provided by an IRE generator.

Example 17. The method according to example 12, wherein the ablation energy comprises radiofrequency (RF) energy provided by an IRE generator.

Example 18. The method according to example 12, wherein the lumen is one of a renal artery and a renal vein of the human subject.

Example 19. A method, comprising:

providing an insertion probe (24), having a distal end configured for insertion into a lumen of a human subject;
extending a resilient tube (32C) distally from the distal end of the insertion probe, the resilient tube having, when unconstrained, a helical shape having no more than a single spiral and an axis that is collinear with a longitudinal axis of the distal end of the insertion probe; and
fixedly attaching a plurality of electrodes (50), configured to transfer ablation energy to the human subject, to the resilient tube,.

Example 20. The method according to example 19, wherein an outer diameter of the helical shape is 5 mm.

Example 21. A method, comprising:

providing an insertion probe (24), having a distal end configured for insertion into a lumen of a human subject;
extending a resilient tube (32D) distally from the distal end of the insertion probe, the resilient tube having, when unconstrained, a linear shape having an axis that is collinear with a longitudinal axis of the distal end of the insertion probe; and
fixedly attaching a plurality of electrodes, configured to transfer ablation energy to the human subject, to the resilient tube.

Example 22. The method according to example 21, wherein an outer diameter of the linear shape is in a range from 1 mm to 3 mm.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

**Claims**

1. A catheter, comprising:

an insertion probe, having a distal end configured for insertion into a lumen of a human subject;
a resilient tube, extending distally from the distal end of the insertion probe and having, when unconstrained, a planar serpentine shape contained within a plane that contains a longitudinal axis of the distal end of the insertion probe; and
a plurality of electrodes, fixedly attached to the resilient tube, configured to transfer ablation energy to the human subject.

2. A catheter, comprising:

an insertion probe, having a distal end configured for insertion into a lumen of a human subject;

a resilient tube, extending distally from the distal end of the insertion probe and having, when unconstrained, a helical shape having no more than a single spiral and an axis that is collinear with a longitudinal axis of the distal end of the insertion probe; and
a plurality of electrodes, fixedly attached to the resilient tube, configured to transfer ablation energy to the human subject.

3. A catheter, comprising:

an insertion probe, having a distal end configured for insertion into a lumen of a human subject;
a resilient tube, extending distally from the distal end of the insertion probe and having, when unconstrained, a linear shape having an axis that is collinear with a longitudinal axis of the distal end of the insertion probe; and
a plurality of electrodes, fixedly attached to the resilient tube, configured to transfer ablation energy to the human subject.

4. A method, comprising:

providing an insertion probe, having a distal end configured for insertion into a lumen of a human subject;
extending a resilient tube distally from the distal end of the insertion probe, the resilient tube having, when unconstrained, a planar serpentine shape contained within a plane that contains a longitudinal axis of the distal end of the insertion probe; and
fixedly attaching a plurality of electrodes, configured to transfer ablation energy to the human subject, to the resilient tube.

5. The catheter according to claim 1 or the method according to claim 4, wherein the resilient tube comprises a plurality of straight elements coupled together as a single linear entity.

6. The catheter according to claim 1 or the method according to claim 4, wherein the resilient tube comprises a plurality of curvilinear elements coupled together as a single linear entity.

7. The catheter according to claim 1 or the method according to claim 4, wherein the planar serpentine shape has a peak-peak value between 2 mm and 6 mm.

8. The catheter according to claim 1 or the method according to claim 4, wherein the ablation energy comprises irreversible electroporation (IRE) energy provided by an IRE generator.

9. The catheter according to claim 1 or the method according to claim 4, wherein the ablation energy comprises radiofrequency (RF) energy provided by an IRE generator.

10. The catheter according to claim 1 or the method according to claim 4, wherein the lumen is one of a renal artery and a renal vein of the human subject.

11. A method, comprising:

providing an insertion probe, having a distal end configured for insertion into a lumen of a human subject;
extending a resilient tube distally from the distal end of the insertion probe, the resilient tube having, when unconstrained, a helical shape having no more than a single spiral and an axis that is collinear with a longitudinal axis of the distal end of the insertion probe; and
fixedly attaching a plurality of electrodes, configured to transfer ablation energy to the human subject, to the resilient tube,.

12. The catheter according to claim 2 or the method according to claim 11, wherein an outer diameter of the helical shape is 5 mm.

13. A method, comprising:

providing an insertion probe, having a distal end configured for insertion into a lumen of a human subject;
extending a resilient tube distally from the distal end of the insertion probe, the resilient tube having, when unconstrained, a linear shape having an axis that is collinear with a longitudinal axis of the distal end of the insertion probe; and

fixedly attaching a plurality of electrodes, configured to transfer ablation energy to the human subject, to the resilient tube.

14. The catheter according to claim 3 or the method according to claim 13, wherein an outer diameter of the linear shape is in a range from 1 mm to 3 mm.

FIG. 1

FIG. 2A

FIG. 2B

FIG. 2C

FIG. 2D

Europäisches
Patentamt

European
Patent Office

Office européen
des brevets

# EUROPEAN SEARCH REPORT

**Application Number**

EP 22 18 9404

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/044312 A1 (ARGA MEDTECH SA [CH]) 11 March 2021 (2021-03-11) | 2,3,7-9, 11-14 | INV. A61B18/12 |
| Y | * paragraphs [0288], [0309] – [0322]; figures 61-64, 69-82 * | 10 | A61B18/14 A61B18/00 |
| | ----- | | |
| X | US 2009/125009 A1 (ZIKORUS ARTHUR W [US] ET AL) 14 May 2009 (2009-05-14) | 1,4,6,7, 12,14 | |
| Y | * paragraphs [0288], [0309] – [0322]; figures 61-64,69-82 * | 5,10 | |
| | ----- | | |
| X | US 5 782 828 A (CHEN PETER CHENG [US] ET AL) 21 July 1998 (1998-07-21) | 1,4,6,7, 12,14 | |
| A | * figure 1 * | 5 | |
| | ----- | | |
| Y | US 2013/253504 A1 (FANG ITZHAK [US]) 26 September 2013 (2013-09-26) | 5 | |
| A | * paragraph [0072]; figures 13A, 13C,13D * | 7,12,14 | |
| | ----- | | |

**TECHNICAL FIELDS SEARCHED (IPC)**

A61B

The present search report has been drawn up for all claims

| Place of search | Date of completion of the search | Examiner |
|---|---|---|
| The Hague | 24 December 2022 | Husselin, Stephane |

EPO FORM 1503 03.82 (P04C01)

## ANNEX TO THE EUROPEAN SEARCH REPORT
## ON EUROPEAN PATENT APPLICATION NO.

EP 22 18 9404

This annex lists the patent family members relating to the patent documents cited in the above-mentioned European search report.
The members are as contained in the European Patent Office EDP file on
The European Patent Office is in no way liable for these particulars which are merely given for the purpose of information.

24-12-2022

| Patent document cited in search report | Publication date | Patent family member(s) | | Publication date |
|---|---|---|---|---|
| WO 2021044312 A1 | 11-03-2021 | CA | 3150063 A1 | 11-03-2021 |
| | | CA | 3150072 A1 | 11-03-2021 |
| | | CN | 114554988 A | 27-05-2022 |
| | | CN | 114641245 A | 17-06-2022 |
| | | EP | 4025130 A1 | 13-07-2022 |
| | | EP | 4025145 A1 | 13-07-2022 |
| | | JP | 2022546736 A | 07-11-2022 |
| | | JP | 2022547096 A | 10-11-2022 |
| | | US | 2022183752 A1 | 16-06-2022 |
| | | US | 2022211427 A1 | 07-07-2022 |
| | | WO | 2021044310 A1 | 11-03-2021 |
| | | WO | 2021044312 A1 | 11-03-2021 |
| US 2009125009 A1 | 14-05-2009 | EP | 1796568 A1 | 20-06-2007 |
| | | US | 2006085054 A1 | 20-04-2006 |
| | | US | 2009125009 A1 | 14-05-2009 |
| | | WO | 2006031541 A1 | 23-03-2006 |
| US 5782828 A | 21-07-1998 | NONE | | |
| US 2013253504 A1 | 26-09-2013 | AU | 2013201681 A1 | 10-10-2013 |
| | | CA | 2809464 A1 | 21-09-2013 |
| | | CN | 103315806 A | 25-09-2013 |
| | | EP | 2641556 A1 | 25-09-2013 |
| | | EP | 3659538 A1 | 03-06-2020 |
| | | IL | 224948 A | 30-04-2017 |
| | | JP | 6165475 B2 | 19-07-2017 |
| | | JP | 2013192948 A | 30-09-2013 |
| | | RU | 2013112613 A | 27-09-2014 |
| | | US | 2013253504 A1 | 26-09-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63231639
- US 8456182 B, Bar-Tal
- US 6690963 B, Ben-Haim
- US 701989